# EUROPEAN PATENT SPECIFICATION

(11) **EP 2 273 926 B1**
(45) Date of publication and mention of the grant of the patent: **23.01.2013**
(21) Application number: 09732564.1
(22) Date of filing: 17.04.2009
(51) Int. Cl.: A61B 10/02

(54) **A PRACTICAL AND SAFE NEEDLE BIOPSY DEVICE**
PRAKTISCHE UND SICHERE NADELBIOPSIEVORRICHTUNG
DISPOSITIF DE BIOPSIE À AIGUILLES PRATIQUE ET SANS RISQUE

(30) Priority: 18.04.2008 TR 200802738
(43) Date of publication of application: 19.01.2011
(73) Proprietor: Eren, Orhan, 55100 Samsun (TR); Ozturk, Ali Ozkan, 55100 Samsun (TR)
(72) Inventor: EREN, Orhan, 55100 SAMSUN (TR)
(74) Representative: Dericioglu, Ekin
(86) International application number: PCT/TR2009/000051
(87) International publication number: WO 2009/128796

(56) References cited:
- EP-A- 1 266 624
- EP-A- 1 889 572
- WO-A-2005/063126
- WO-A-2006/136588
- WO-A-2007/009901
- WO-A-2007/110812
- US-A1- 2005 080 355

## Description

### Technical Field

The invention relates to biopsy needles which enable to take soft pieces of tissue and/or cystic fluids, tissue aspirates, and blood materials from the organs in the human body for determining the reasons of anomalous nodules or masses (tumors) in the human body, and act as a medical invasive test procedure.

Through a method that is safe, simple, and far more easier to use, as if simply administering injection, the said invention can provide possibility of administering closed biopsy to intraabdominal organs/masses that are appropriate for closed biopsy or all kinds of tissues that contain such lesions as LAP; particularly thyroid, breast, lymph node, cystic lesion, and cutaneous/subcutaneous lesions; to every medical doctor who has injection experience.

When internal organ biopsy is being administered, having the patient hold his/her breath and continue this retention throughout the duration of biopsy is very important for prevention of complications. The invention relates to a biopsy needle device that minimize complication risk due to its following properties: aim is taken once with the biopsy needle; the biopsy procedure does not include sequential manoeuvres in tru-cut biopsy, which take longer time, are consecutive, and result in longer procedures; it shortens the duration of procedure due to its being very simple; and it enables to realize whether or not the needle is in intravenous position.

### Background of the Invention

There are two types of biopsies: open and closed. Open biopsy: Cutaneous and subcutaneous tissues are opened with a surgical procedure and the biopsy is administered to the relevant organ by a surgical method in which there is visual clue. Due to its requirement of surgical procedure, it is not useful and practical, and it is a highly invasive procedure.

Closed biopsy: It is a method which is administered with the help of needle, directed at taking pieces of tissue from target organs, less invasive, and compared to open biopsies it is used much more often in daily applications.

Needle Aspiration Biopsy: Except bone marrow biopsy which is administered with a thick needle, aspiration biopsies that are administered with a thin needle are among closed biopsies that are used much more often. Thin Needle Aspiration Biopsy (TNAB): It is a method that is administered most frequently to thyroid and lymph nodes, and one of the methods used most frequently. In this method, target lesion in target tissues such as thyroid or breast is reached with the needle and the injection syringe piston is pulled back, thus vacuum is produced inside cell or tissue aspirate containing fluid in the target tissue, and it is vacuumed first into the needle and then into the hollow of the injection syringe. The needle used here is one-piece. Cases where sufficient piece of tissue cannot be taken with the TNAB method and faulty negative results are substantially frequent.

As a Closed Biopsy Method, tru-cut biopsy is used as the second most common method, and it is frequently preferred in breast and renal biopsies. There are gun-shaped types of tru-cut which take biopsy automatically.

Other Biopsy Methods: There are also other biopsy methods such as Seldinger and Abraham, which are respectively directed at liver and pleura biopsies; these methods are not mentioned here.

Insufficiencies of the present tru-cut application method are as follows: their uses require experience and mastery. In this needle biopsy system with an inner needle and a cylindrical cannula with cutting edge which cover the inner needle;
- It can be moved forward toward the target tissue when aspiration inner needle with a cavity at the tip is aligned with the tip of outer cylindrical cutter. For this reason, in order to make an incision, first the inner needle and then the outer needle must be once more made to manoeuvre within the organ.
- When the needle is inserted into the organ, it is necessary to aim not at the target tissue but at a nearer location. This distance between the points that are aimed, which is nearer than the target tissue, and the target point is the same length as the length of inner needle aspiration reservoir (a few mm.). Even achieving this distance requires estimation and mastery, and it also includes margin of error of not reaching the target tissue.
- When the inner needle is pushed inside, one must aim once more so that the target tissue can be reached.
- The biopsy is taken by carrying out an incision procedure where outer needle cylindrical cutting tube is pushed.
- Finally, the needle is pulled out. This present tru-cut biopsy method also requires a certain education and mastery.

There are many patent applications pertaining to biopsy needle devices. Among these applications, there is a patent application with number EP1903945A1. In this application, the aspiration inner tip goes out during the biopsy. It differs from the tru-cut in that the inner aspiration needle goes out and then it is retracted; that is, it is like a modified tru-cut system and the biopsy material is collected in the cavity within the inner needle. In the injection biopsy, which is our invention, there is a cutting inner needle, and there are no back and forth displacement manoeuvres in the incision procedure that is done when in the target tissue. The EP1903945A1 is not in form of an injection syringe and it does not include combination of aspiration.

On the other hand, in the patent application JP2007054449A, outer lumen is rotated with a rope style material connected to the needle, and material is taken with the tip of the needle; it seems to be a rudimentary and ineffective method. Also, it is a semi-active system that is very different from our invention.

On the other hand, in the patent application with number W02007110812A2, there is a system which carries out coaxial incision with the help of vacuum. Aspiration notch on the inner lumen go out of the system and carry out vacuuming action, and incision is made with cutting cylinder during its re-entry. Incision method and design are different, and it does not include combination of aspiration.

Document US-A-2005/080355 is also considered relevant.

### Description of the Invention

Most important purpose of the invention is taking of the incision biopsy by enabling incision not with outer cannula but with inner cutting needle, through a simple injection manoeuvre, by connecting inner and outer needles in the present tru-cut biopsy system with body and piston of the standard injection syringes. It includes devices, where the inner cutting needle may have one-way back and forth movements in directions of a-b as well as devices which have movements in directions of left-right or spiral movements, as variations. As an addition to this purpose of incision biopsy, another purpose is development of an injection-incision biopsy device that is practical and that can be safely applied to all soft tissue biopsies by allowing aspiration of fluid inside the mass, in case there are any, by establishing, through conveyor channel and outlet, a relationship between inlet on the inner needle and hollow in the injection syringe body.

Another advantage of the invention is that it enables aiming once at the target tissue and taking sufficient amount of tissue piece from the target tissue.

Another purpose of the invention is to allow its use for cystic lesions, because, due to existence of hollow channel at one edge of the inner needle, it can be used in aspiration of the fluid inside the cyst if the target tissue is cystic, that is, if it is a fluid-containing structure. With this property, it provides an advantage in taking of biopsy from cystic nodules that are frequently observed, particularly, in goiter patients.

Another purpose of the invention is that due to its being single incision biopsy that also allow thin needle aspiration system, it provides opportunity to know that the needle is in intravenous position, which is an unwanted complication of the target tissue that is reached during the biopsy procedure. In such a situation where the needle is in intravenous position, such a possibility of complication that increase the risk of haemorrhage will be known before making an incision, because blood will enter into the syringe during the action of pulling back the piston, which is the move that takes place before the incision manoeuvre, and the risk of haemorrhage, which is the most feared aspect of closed biopsy method, will be reduced.

From the viewpoint of reducing the risk of haemorrhage, another advantage of the invention is, due to entry of arterial blood into the injection syringe with pressure, in a flushing manner, particularly when the needle is in intravenous position prior to the biopsy, it is known that the needle is in intravenous position before making the incision; and reduction of the risk of heavy-fatal arterial haemorrhage, which is most feared complication in biopsies.

Another purpose of the invention is reduction of reports of insufficient material in pathological examination reports, due to the invention's being a combined higher technique that can be also used in taking incision biopsy together with the TNAB method, when it is used instead of the TNAB, particularly in such lesions as thyroid and lymph nodes, nodular, etc. where the biopsy is administered with the TNAB.

Following method may be used in order to fulfil purposes and advantages explained above: positioning of the cutting inner needle that is connected to the piston of the injection syringe in hollow of the outer needle that is connected to the body of the said injection syringe and carrying out the incision procedure of the target tissue with the cutting inner needle; incision of the tissue with tip of the cutting inner needle and taking it into the aspiration reservoir that is between the outer surface of the outer needle and the inner cutting edge, by means of opening of the notch at the tip of the outer needle of the cutting inner needle and occurring of both passive filling and vacuum effect with the opening of the notch through retraction of the said moveable cutting inner needle by means of the outer needle that is inserted into the target tissue in the human body.

In addition to the incision biopsy explained above, in order to fulfil the purpose of aspiration, it contains a connection head that has a cavity that is formed on the cutting inner.needle and an inlet that is formed at the cutting edge of the cutting inner needle; for transfer of tissue aspirate, cystic fluid, and blood elements that are first taken into conveying space; into the injection syringe hollow of the injection syringe body by means of the cutting inner needle.

### Brief Description of the Figures

In order to fully understand the construct of the present invention and its advantages due to additional elements, it must be evaluated with the figures that are explained below.
Figure-1; Complete two-dimensional side assembly view of the biopsy needle device, when the inner needle positioned in the outer needle is retracted in direction b.
Figure-2; Two-dimensional side view of the outer needle of the biopsy needle device.
Figure-3; Two-dimensional side view of the inner needle of the biopsy needle device.
Figure-4; Complete two-dimensional side assembly view of the biopsy needle device, when the inner needle positioned in the outer needle is moved forward in direction a.
Figure-5; Two-dimensional disassembly view of the outer needle when it is separated from the body of the injection syringe.
Figure-6; Two-dimensional disassembly view of the inner needle when it is separated from the moveable body of the injection syringe.

### Reference Numbers

10- Outer needle (cannula)
11- Hollow
12- Needle tip
13- Notch
14- Needle connection head
15- Head connection surface
16- Injection syringe body
17- Hollow of the injection syringe
18- Impermeability element
19- Tissue aspiration reservoir
20- Cutting inner needle
21- Cutting edge
22- Inlet
23- Conveying space
23.1- Transition channel
24- Moveable piston
25- Pressure surface
26- Connection head
27- Outlet

### Detailed Description of the Invention

In respect of its structure pertaining to the present art, the invention contains an injection syringe body (16), injection syringe hollow (17) that is formed inside the said body (16) and impermeability element (18) that is positioned in this hollow (17), moveable piston (24) and pressure surface (25) that is formed on this piston (24).

In order to ensure aiming in one go and easy use of the biopsy needle device, these steps include injection incision procedure which consists of the following steps: positioning of the cutting inner needle (20) in hollow (11) of the outer needle (10) that is connected to the body (16) of the said injection syringe and carrying out the incision procedure of the target tissue with the cutting inner needle (20); opening of the notch (13) at the tip of the outer needle (10) with the cutting inner needle (20) through retraction of the said moveable body (24) with its movement in direction b (Figure-6) by means of the outer needle (10) that is inserted into the target tissue in the human body and occurrence of both passive filling and vacuum effect with the opening of the notch (13) and taking of the tissue into the tissue aspiration reservoir (19) by means of the cutting edge (21) inlet.

In addition to the incision biopsy explained above, in order to carry out the aspiration procedure, it contains a connection head (26) that has an outlet (27) that is formed on the cutting inner needle (20) and an inlet (22) that is formed at the cutting edge (21) of the cutting inner needle (20); for transfer, through conveying space (23), of such samples as cystic fluid, tissue aspirate, and blood elements taken with the vacuum effect that is created by opening of the cutting inner needle (20) into the injection syringe hollow (17) of the said injection syringe body (16). For making this aspiration procedure easier, the system can be integrated with valve system that enables unidirectional fluid passage.

Usage and manner of working of the device is as follows:
In this device, the most important component and the most important difference compared to the existing devices is that the procedure of incision and taking in of the tissue is made by the cutting needle (20), and the cutting needle (20) which assumes this role is positioned inside the outer needle (10). On the other hand, another important aspect is that the outer needle (10) takes on the role of insertion into the patient's body and after being locked into the target it carries out bedding function for rectilinear motion of the cutting needle (20) (in directions a - b) by remaining in a fixed position inside the hollow (11), and it provides the tissue aspiration reservoir (19) for the notch (13) that it contains at its terminal side and the target piece of tissue.

In respect of its position in figure-4, the biopsy needle device is inserted into the patient's body in closed state. In closed position, the cutting needle (20) closes aperture space of the notch (13). When the moveable body (24) is retracted (in direction b) by means of the pressure surface (25), it begins to open the aperture space of the notch (13). (See figure-1) When the retraction process is continuing, due to vacuuming, the piece of tissue is first directed into the conveying space (23) of the cutting needle (20); and thus, filling of such materials as cystic fluid, blood, and tissue aspirate, in case there are any, into the hollow of the injection syringe (17) is achieved. Following-this, the moveable piston (24) is pushed forward, the piece of tissue that enter into the notch (13) is incised and taken into the tissue aspiration reservoir (19), and when the outer needle notch is in closed position, where is it closed by the inner needle as shown in figure-4, the needle is completely pulled out of the body. The said notch (13) may have different geometrical forms and sizes. It may contain more than one notch (13). Needle tip (12) of the outer needle (10) may be open or closed.

As mentioned above, the piece of tissue that is taken from the patient is first directed toward the tissue aspiration reservoir (19) of the cutting needle (20). If there are liquid/semi-liquid fluids such as cystic fluids, tissue aspirates, and blood elements here, these are made to fill into the conveying space (23) by means of the inlet (22) that is formed at the terminal side of the cutting needle (20), and from there into the hollow of injection syringe (17) through the outlet (19) and the connection head (26). The conveying space (23) also has a transition channel (23.1). Preferably, it has the appearance of a crescent, and preferably it can be produced in this shape. (See detail a)

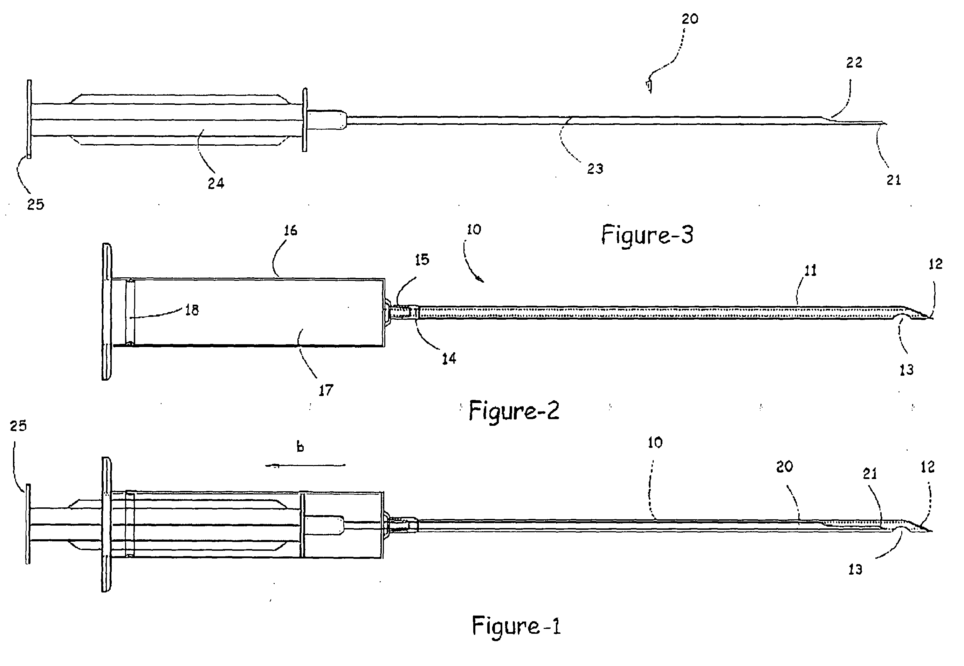

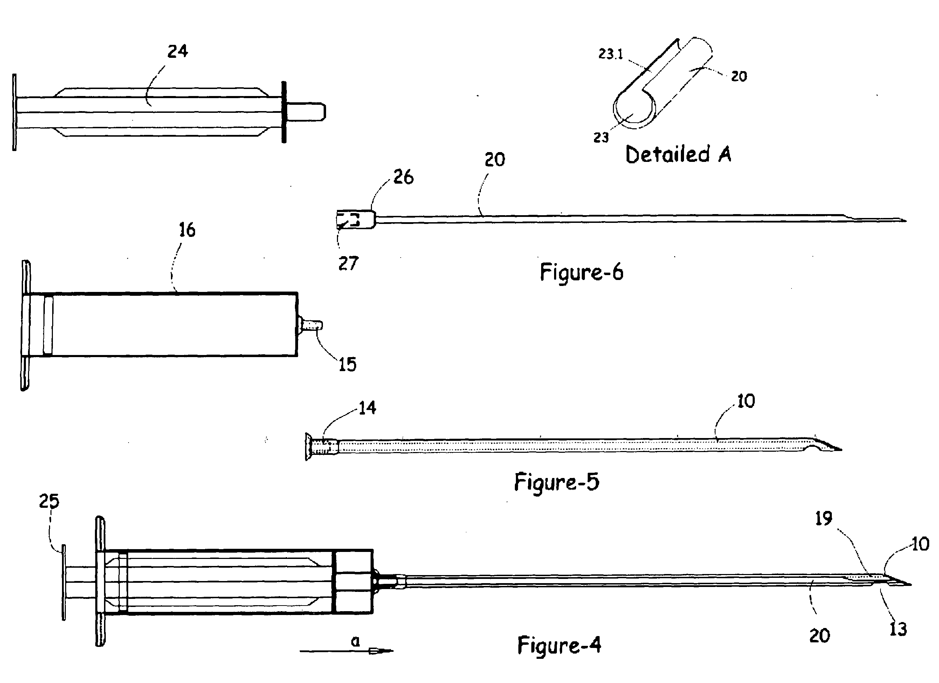

## Claims

1. A needle biopsy device adapted to take of incision biopsy and/or tissue aspirate, cystic fluid, blood material from anomalous nodules or masses (tumors) in the human body comprising
- an injection syringe body (16),
- a hollow of the injection syringe (17) that is formed inside the injection syringe body (16),
- an impermeability element (18) that is positioned in the hollow of the injection syringe (17),
- a moveable piston (24),
- a pressure surface (25) that is formed on the moveable piston (24),
- an outer needle (10) one tip of which is attached to the injection syringe body (16),
- a hollow (11) that is formed inside the outer needle (10),
- a needle tip (12) that is located at the outer needle (10) tip which is not attached to the injection syringe body (16),
- a needle connection head (14) that enables the outer needle (10) to be attached to the injection syringe body (16),
- a head connection surface (15) that is located at the part of the injection syringe body (16) whereto the outer needle (10) is attached, and enables the needle connection head (14) to be fixed to the injection syringe body (16),
- a notch (13) that is located on the outer needle (10) and enables the mass or the nodule from which incision biopsy and/or tissue aspirate, cystic fluid, blood material will be taken of to enter the hollow (11) inside the outer needle (10),
- a cutting inner needle (20) that is located at the hollow (11) inside the outer needle (10) and fixed to the moveable piston (24) tip which has no pressure surface (25),
- cutting edge (21) that is located at the cutting inner needle (20) tip which is distal from the injection syringe body (16),
- an inlet (22) that is formed at the cutting edge (21) of the cutting inner needle (20) and enables liquid/semi-liquid fluids such as cystic fluids, tissue aspirates, blood elements to be taken in from the cutting edge (21) of the cutting inner needle (20),
- a conveying space (23) that is formed in the cutting inner needle (20) and enables liquid/semi-liquid fluids such as cystic fluids, tissue aspirates, blood elements received from the inlet (22) to be transferred towards the injection syringe body (16) along the cutting inner needle (20),
- a transition channel (23.1) that is formed in the cutting inner needle (20),
- a connection head (26) that is located on the cutting inner needle (20) side which is fixed to the moveable piston (24), and serves to fix the cutting inner needle (20) to the moveable piston (24) and
an outlet (27) that enables liquid/semi-liquid fluids such as cystic fluids, tissue aspirates, blood elements to be removed from the conveying space (23).

2. Device according to claim 1; wherein, it contains a connection head (26) that has an outlet (27) and an inlet (22) that is formed on the cutting inner needle (20); for transfer of such samples as tissue aspirate, cystic fluid, and blood elements taken by means of the cutting inner needle (20) and the notch (13) into the injection syringe hollow (17) of the said injection syringe body (16).

3. Device according to claims 1 and 2; wherein, it contains an inlet (22) that is connected with the conveying space on the inner needle, and formed at the cutting edge (21) of the said cutting inner needle (20).

4. Device according to claims 1, 2, and 3; wherein, it contains the transition channel (23.1) that is formed on the said cutting inner needle (20).

5. A needle biopsy device according to any one of the preceding claims, **characterized by** a valve which enables one-way fluid transfer in order to facilitate aspiration procedure.

## Patentansprüche

1. Eine Nadelbiopsie-Vorrichtung, die daran angepasst ist, Inzisionsbiopsie und/oder Gewebe-Absaugung, Zystenflüssigkeit, Blut-Material aus abnormen Knötchen oder Massen (Tumoren) im menschlichen Körper zu entnehmen, **umfassend,**
- einen Injektionsspritzenkörper (16),
- einen Hohlraum der Injektionsspritze (17), die innerhalb des Injektionsspritzenkörpers (16) gebildet ist,
- ein Dichtungselement (18), das in dem Hohlraum der Injektionsspritze (17) positioniert ist,
- einen beweglichen Kolben (24),
- eine Druckoberfläche (25), welche auf dem beweglichen Kolben (24) gebildet ist,
- eine äußere Nadel (10), deren eine Ende an den Injektionsspritzenkörper (16) angefügt ist,
- einen Hohlraum (11), der innerhalb der äußeren Nadel (10) gebildet ist,
- eine Nadelspitze (12), die an der äußeren Nadel (10) angeordnet ist, welche an den Injektionsspritzenkörper (16) nicht angefügt ist,
- einen Nadelanschluss-Kopf (14), welcher es ermöglicht, dass die äußere Nadel (10) an den Injektionsspritzenkörper (16) befestigt wird,
- eine Kopfanschlussoberfläche (15), welche an demjenigen Teil des Injektionsspritzenkörpers angeordnet ist, an das die äußere Nadel (10) angefügt wurde und welche es ermöglicht den Nadelanschlusskopf (14) an den Injektionsspritzenkörper (16) zu befestigen,
- eine Kerbe (13), die an der äußeren Nadel (10) angeordnet ist und die es ermöglicht, dass die Masse oder Knötchen, aus welchen Inzisionsbiopsie und/oder Gewebe-Absaugung, Zystenflüssigkeit, Blut-Material zu entnehmen ist, in den Hohlraum (11) innerhalb der äußeren Nadel (10) eintritt,
- eine schneidende innere Nadel (20), die an dem Hohlraum (11) innerhalb der äußeren Nadle (10) angeordnet ist und am beweglichen Kolben (24), der keine Druckoberfläche (25) aufweist, befestigt ist,
- Schneide-Kante (21), die an der inneren Schneide-Nadelspitze (20) angeordnet wurde, welche fern von dem Injektionsspritzenkörper (16) ist,
- einen Einlass (22), der an der Schneide-Kante (21) der schneidenden inneren Nadel (20) gebildet ist und welcher es ermöglicht, das flüssige/halbflüssige Medium wie zystische Flüssigkeiten, Gewebe-Absaugungen, Blutelemente von der Schneide-Kante (21) der schneidenden inneren Nadel (20) zu entnehmen,
- einen Förderraum (23), der in der schneidenden inneren Nadel (20) gebildet ist und es ermöglicht, das flüssige/halbflüssige Medium wie zystische Flüssigkeiten, Gewebe-Absaugungen, Blutelemente, welche aus dem Einlass (22) hereingenommen sind, entlang der schneidenden inneren Nadel (20) auf den Injektionsspritzenbody (16) zu übertragen,
- einen Überleitungskanal (23.1), der in der schneidenden inneren Nadel (20) gebildet ist,
- einen Anschlusskopf (26) der auf der schneidenden inneren Nadel (20) angeordnet ist, welcher an den bewegbaren Kolben befestigt wurde und dazu dient, die schneidenden inneren Nadel (20) an den bewegbaren Kolben (24) zu befestigen und
- einen Auslass (27), der es ermöglicht, dass das flüssige/halbflüssige Medium wie zystische Flüssigkeiten, Gewebe-Absaugungen, Blutelemente aus dem Förderraum entnommen werden.

2. Vorrichtung nach Anspruch 1, wobei sie einen Anschlusskopf (26) aufweist, der einen Auslass (27) sowie einen Einlass (22) umfaßt, welche auf der schneidenden inneren Nadel (20) gebildet sind um die mit Hilfe von schneidender innerer Nadel (20) und der Kerbe (13) entnommenen zystische Flüssigkeiten, Gewebe-Absaugungen und Blutelemente in den Hohlraum der Injektionsspritze zu überleiten.

3. Vorrichtung nach Ansprüchen 1 und 2, wobei sie einen einlass (22) aufweist, der mit dem Förderraum auf der inneren Nadel verbindet ist und an der Schneide-Kante (21) der genannten inneren Nadel (20) ausgebildet ist.

4. Vorrichtung nach Ansprüchen 1, 2 und 3, wobei sie einen Überleitungskanal (23.1) aufweist, welcher an der genannten schneidenden inneren Nadel (20) ausgebildet ist.

5. Eine Nadelbiopsie-Vorrichtung nach einem der vorangehenden Ansprüchen, **gekennzeichnet durch** ein Ventil, das zur Erleichterung des Absaugvorganges eine einseitig gerichtete Überleitung der Flüssigkeit ermöglicht.

## Revendications

1. Un dispositif de ponction-biopsie adapté à prendre biopsie-exérèse et/ou aspiration tissulaire, liquide kystique, nodules d'anomalie, ou masses (tumeurs) du corps humain **comportant :**
- Un corps de seringue d'injection (16),
- Une cavité (17) de la seringue d'injection formée à l'intérieur du corps de seringue d'injection,
- Un élément imperméable (18) situé dans la cavité (17) de la seringue d'injection,
- Un piston mobile (24),
- Une surface de pression (25) formée sur le piston mobile,
- Une aiguille extérieure (10) dont une extrémité est liée au corps de seringue d'injection,
- Une cavité (11) formée à l'intérieur de l'aiguille extérieure (10),
- Une pointe d'aiguille (12) se trouvant sur la pointe de l'aiguille extérieure (10) n'étant pas connectée au corps de seringue d'injection (16),
- Une tête de connexion d'aiguille (14) assurant la connexion de l'aiguille extérieure (10) au corps de seringue d'injection (16),
- Une surface de connexion de tête (15) se trouvant sur le corps de seringue d'injection auquel est liée l'aiguille extérieure et assurant la fixation de la tête de connexion d'aiguille (14) au corps de seringue d'injection,
- Une coche (13) se trouvant sur l'aiguille extérieure et assurant l'entrée de la masse et le nodule duquel on prendra biopsie-exérèse et/ou aspiration tissulaire, liquide kystique, et matériau sanguin dans la cavité à l'intérieur de l'aiguille extérieur (10),
- Une aiguille intérieure incisive (20) se trouvant dans la cavité à l'intérieure de l'aiguille extérieur (10) et fixée sur l'extrémité de piston mobile (24) n'ayant pas une surface de pression (25),
- Un bord incisif (21) se trouvant sur l'extrémité de l'aiguille intérieure incisive (20) situé loin du corps de seringue d'injection,
- Une entrée (22) pratiquée dans le bord incisif (21) de l'aiguille intérieure incisive et assurant la réception des liquides/semi-liquides tels que liquides kystiques, aspirations tissulaires, et matériaux sanguins du bord de l'aiguille incisive (20),
- Une surface porteuse (23) formée dans l'aiguille intérieure incisive (20) et assurant le transfert des liquides/semi-liquides tels que liquides kystiques, aspirations tissulaires, et matériaux sanguins pris de l'entrée (22) vers le corps de seringue d'injection le long de l'aiguille intérieure incisive (20),
- Un canal de passage (23.1) formé dans l'aiguille intérieure incisive (20),
- Une tête de connexion (26) se trouvant à côté de l'aiguille intérieure fixé au piston mobile (24) et assurant la fixation de l'aiguille intérieure incisive (20) au piston mobile (24),
- Une sortie (27) assurant l'extraction des liquides/semi-liquides tels que liquide kystique, aspiration tissulaire, et matériaux sanguins de la surface porteuse (23).

2. Dispositif selon la revendication 1, comportant une tête de connexion possédant une sortie (27) et une entrée pratiquées sur l'aiguille intérieure incisive ; et assurant la prise des échantillons des matériaux tels que aspiration tissulaire, liquide kystique, et éléments sanguins par l'aiguille intérieure incisive (20) et la coche (13) et leur transfert à la cavité de seringue d'injection (17) dudit corps de seringue d'injection (16).

3. Dispositif selon les revendications 1 et 2, comportant une entrée (22) liée à la surface porteuse sur l'aiguille intérieure et formée à côté du bord incisif (21) de l'aiguille intérieure incisive.

4. Dispositif selon les revendications 1, 2, et 3, comportant un canal de passage (23.1) formé sur ladite aiguille intérieure incisive (20).

5. Un dispositif de ponction-biopsie selon l'une quelconque des revendications précédentes, **caractérisé par** une valve assurant le transfert unidirectionnel liquide afin de faciliter la procédure d'aspiration.
